# EUROPEAN PATENT SPECIFICATION

(11) **EP 3 541 974 B1**
(45) Date of publication and mention of the grant of the patent: **16.12.2020**
(21) Application number: 16837985.7
(22) Date of filing: 18.11.2016
(51) Int. Cl.: D01H 13/16, B65H 63/028, G01N 33/36

(54) **OPTICAL CONTROL SYSTEM FOR SPINNING FRAMES**
OPTISCHE SENSOREINRICHTUNG FÜR EINE SPINNMASCHINE
SYSTÈME DE CONTRÔLE OPTIQUE POUR UN MÉTIER À FILER

(43) Date of publication of application: 25.09.2019
(73) Proprietor: Pinter Fa.Ni S.r.l., 36072 Chiampo (VI) (IT)
(72) Inventor: BAESSATO, Feror, 36072 Chiampo (VI) (IT); COVOLO, Sergio, 36030 Sarcedo (VI) (IT)
(74) Representative: Ziliotto, Tiziano
(86) International application number: PCT/IT2016/000272
(87) International publication number: WO 2018/092162

(56) References cited:
- EP-A1- 2 042 877
- DE-A1- 3 536 889
- DE-A1- 4 233 285
- FR-A- 1 358 637
- US-A- 5 017 797
- US-A1- 2009 141 277

## Description

### FIELD OF APPLICATION OF THE INVENTION

The present invention concerns a control device for spinning frames.

### STATE OF THE ART

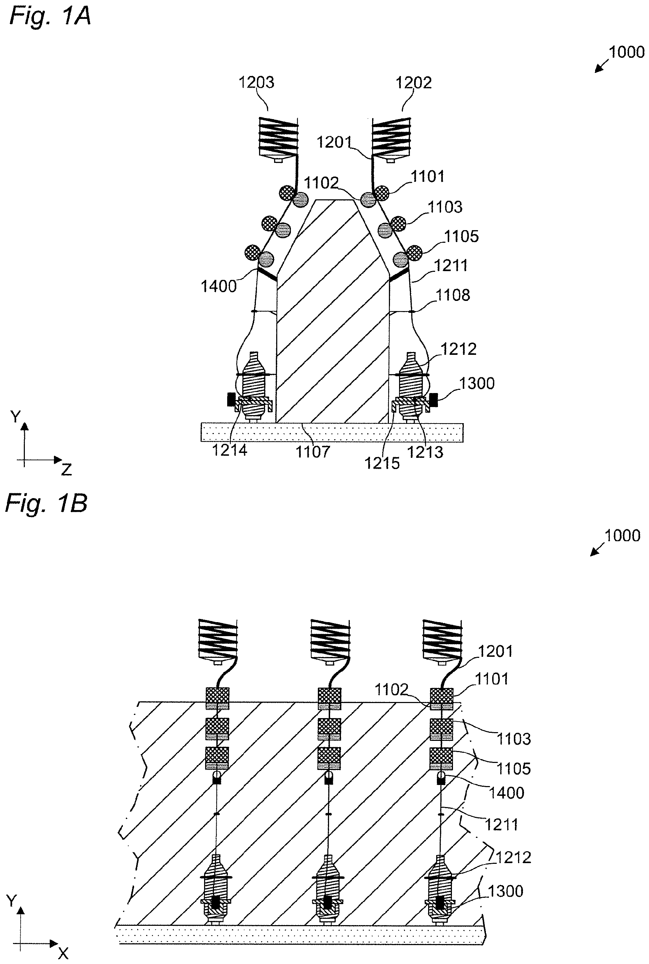

Figure 1A shows a schematic sectional view of elements of a spinning frame 1000 known in the art. Figure 1B shows a schematic front view of elements of the spinning frame 1000.

The spinning frame 1000 substantially comprises a body 1107 on which a plurality of spindles are located. The number of spindles can be very high, in fact spinning frames provided with more than 1800 spindles are known. Each spindle comprises a plurality of drawing rollers 1101-1106, generally three pairs 1101 and 1102, 1103 and 1104, 1105 and 1106, which draw a roving 1201 arriving from a bobbin 1202. The drawing process is carried out by making the three pairs of drawing rollers 1101-1106 rotate at different speeds. In particular, it is sufficient that at least one first pair, for example the rollers 1101-1102, rotates at a lower speed than a second pair, for example the rollers 1105-1106, placed downstream of the first pair in the direction of movement of the roving 1201. Figure 1A schematically shows two spindles for drawing, independently of each other, the rovings arriving from the bobbins 1202 and 1203. Figure 1B schematically shows three spindles.

Drawing the roving 1201 results in a yarn 1211 which, when leaving the last pair of rollers 1105-1106, is twisted through the twisting movement transmitted by the successive rotation around the spool 1212. In particular, possibly after passing through a yarn guide lappet 1108, the yarn 1211 is rolled on a spool or spindle 1212 through the rotation of a ring traveller 1213 around the spool 1212. The yarn produced can have different characteristics in terms of uniformity and resistance, and have more interlocked surface fibres as a result of the use of double-strand yarns obtained directly from the ring spinning frame with suitable adaptations, as described here below.

During the spinning process, it is useful to check that the yarn 1211 is actually wound on its spool 1212 and/or that it is wound with its twisting and titre characteristics. For example, titre by length means the ratio between length and weight: according to this titration system, the higher is the titre the finer, that is, the thinner is the yarn.

In particular, it is useful to check one or more of the following parameters. For example, (i) the presence of the yarn 1211, that is, it is advisable to make sure that the yarn 1211 is not broken and is actually being wound on its spool 1212. This check makes it possible to avoid any waste of material since, in case of breakage of the yarn 1211, the roving 1201 advances but is not twisted, its spool 1212 turns without winding the yarn, with a consequent waste of energy, time, material and with the risk of creating problems to the adjacent spindles. It is also interesting, for example, (ii) to check the twisting of the yarn, that is, to make sure that the yarn 1211 leaving the last pair of drawing rollers 1104, 1105 is correctly twisted. For this purpose, it is necessary to check the revolutions of the ring traveller 1213.

According to the state of the art, this check is performed by a sensor 1300, of the inductive and/or optical type, which detects the passage of the ring traveller 1213 during the winding of the yarn. In particular, inductive sensors detect the electrical signal induced in their bobbin by the variation of the magnetic field produced by the passage of the ring traveller 1213 during the winding of the yarn on the spool. Optical sensors are based on the emission of an infrared optical signal and on the analysis of the reflected signal altered by the passage of the ring traveller 1213. These sensors are placed on the ring rail 1215 in front of the ring 1214 of the spool 1212, occupy a certain space that hinders the introduction of the yarn 1211 in the ring traveller 1213 during the repair of the yarn on the spool 1212 and hinder the maintenance of the ring 1214, for example in terms of centering. Furthermore, the container of the sensor 1300 causes residual materials and fibres, which may cause other faults on the adjacent spindles, to be deposited on the sensor 1300 itself. These sensors 1300 with their electronic components, printed circuits and wiring harnesses are contained and distributed in metal or plastic raceways fixed to the ring rails, raceways which occupy a certain space, thus increasing the lateral dimensions of the spinning frame 1000 (in the direction Z) and making the ring rails and/or the raceways subject to possible impacts against the vehicles and tools used by the operators working along the aisles between the spinning frames. Furthermore, the sensors increase the weight of the ring rail 1215 which must be continuously lifted and lowered in order to wind the yarn 1211 on the spool 1212, with consequent mechanical balancing problems and increased energy consumption.

In particular, magnetic sensors generally use a permanent magnet that produces a considerable attractive force on the ring traveller 1213 and, especially during the starting phase, can cause the yarn 1211 to break preventing the sliding movement of the ring traveller 1213. Optical sensors pose difficulties in terms of setting for the centering and detection of the ring traveller 1213 in front of the ring 1214 and this may make them scarcely reliable. Furthermore, the dispersed fibres and the residual dust resulting from the processing cycles and always present on spinning frames are deposited on the optical parts and affect their performance. Furthermore, other types of sensors are known, which are suited to detect only the presence of the yarn 1211, such as barrier optical sensors, capacitive, piezoelectric, force sensors which however, due to high costs, scarce reliability, difficulty in positioning, are not used on ring spinning frames in which the high number of spindles to be controlled and therefore the high number of sensors to be used make it actually possible to use only sensors that can be grouped and managed in a single set or convenient subsets, in order to reduce costs. Furthermore, the sensors and their answers must be managed in real time in order to effectively control the yarn 1211. In particular, when the sensor detects a tear in the yarn 1211, it is possible to activate a corresponding roving locking device, not illustrated in the drawings, in such a way as to prevent the roving 1201 from becoming twisted around the drawing rollers 1101-1106 and/or to avoid any waste of material.

Furthermore, in the spinning frames where the yarn 1211 is obtained starting from two rovings, known as double yarn or Siro spun yarn frames, it is also useful to be able to check (iii) that the final yarn is made up of two strands. The absence of one of the two yarns affects the characteristics of the desired product, which keeps being wound on the spool, but with one yarn only. This spool must then be recovered by the machine that follows the spinning frame, the spooler, which must unwind the spool, recover the yarn made up of a single strand and eliminate it, wasting material and time. None of the sensors known in the art is capable of detecting the presence of the two yarns in an efficient manner. Control devices according to the prior art are known from documents EP 2 042 877 A1, US 2009/141277 A1, FR 1 358 637 A, US 5 017 797 A and DE 35 36 889 A1, JP H02 74629A.

### SUMMARY OF THE INVENTION

It is thus one object of the present invention to provide a control system for spinning frames having improved characteristics and possibly capable of solving at least one of the problems (i) (ii) (iii) described above.

In particular, according to some embodiments it will be possible to provide an improved control system capable of determining the correct twisting of the yarn based on the vibration frequency of the same.

In general, the present invention is based on the concept according to which it is possible to find the correct twisting of the yarn by measuring its movement and the corresponding vibration frequency.

In this way, in some embodiments it is possible to avoid any direct contact with the yarn and/or the presence of guides and/or forks on which the yarn should be channelled and which may alter the characteristics of the yarn itself.

Furthermore, the overall dimensions of the control device are reduced and the latter can be easily positioned, for example, on the inner side of the spinning frame.

An embodiment of the invention concerns a control system for spinning frames, the spinning frames comprising a plurality of spindles, each spindle comprising first drawing rollers configured in such a way as to draw a first roving and thus obtain a first yarn, the control system comprising a plurality of control devices associable to the plurality of spindles, each control device comprising: an optical movement sensor, a frequency meter, wherein the optical movement sensor is configured so as to measure the movement of the yarn, wherein the frequency meter is configured so as to receive the signal measured by the optical movement sensor and so as to measure at least one oscillation frequency of the yarn.

Owing to this embodiment, it is possible to measure the oscillation frequency of the yarn while at the same time avoiding any physical contact with the yarn. Furthermore, based on the measured frequency value, it is possible to determine some characteristics of the spinning process. In particular, it is possible to determine whether the spinning process is being carried out correctly or whether the yarn is broken. In the cases where there are several yarns joined together, this embodiment makes it possible to determine even if only one of the yarns is missing. Furthermore, it is more generally possible to determine the rotation speed of the yarn on the bobbin, and thus to identify any errors in the rotation of the yarn around the bobbin even in the presence of the yarn.

In some embodiments, the spinning frame may also comprise drawing rollers configured so as to draw a second roving and thus obtain a second yarn, the optical movement sensor can be configured so as to measure the movement of the second yarn and/or of the first and the second yarn combined together, the frequency meter can be configured so as to receive the signal measured by the optical movement sensor and so as to measure at least one oscillation frequency of the second yarn and/or of the first and the second yarn combined together.

Owing to this embodiment, it is possible to use the control device also in spinning frames where the final yarn is constituted by two or more strands.

In some embodiments, the optical movement sensor may comprise an optical generator configured so as to emit a modulated radiation with a predetermined frequency.

Owing to this embodiment, it is possible to measure the movement of the yarn by analysing an amplitude-modulated signal (AM) measured by the optical movement sensor, possibly using known signal analysis techniques.

In some embodiments, the frequency meter may comprise a spurious frequency detector configured so as to determine the presence of at least one spurious frequency in the signal measured by the optical movement sensor.

Owing to this embodiment, it is possible to determine the presence of the spurious frequencies that otherwise may lead to errors in the measurement of the oscillation frequency of the yarn.

Furthermore, the presence of the spurious frequencies can be used to set the device and/or to determine the presence or absence of one or more yarns.

In some embodiments, the spurious frequency detector can be configured so as to reduce and/or increase the modulated radiation power and/or the amplification of the measured signal if the presence of at least one spurious frequency is determined.

Owing to this embodiment, it is possible to modify the power and/or amplification parameters in order to increase the difference in amplitude between the spurious frequency and the yarn oscillation frequency.

According to the invention, the control device comprises a controller connected to the frequency meter, wherein the controller is configured so as to determine the presence of a problem if the oscillation frequency is not included within a predetermined interval (INT).

Owing to this embodiment, it is possible to determine on what spinning points there is a problem, thanks to the measurement of the yarn oscillation frequency.

Furthermore, the information collected from different spindles can be combined so as to define the predetermined interval within which the yarn oscillation frequency can be considered acceptable.

This allows the control system to constantly follow the evolution of the spinning process and to adapt to possible modifications.

Accroding to the invention, the controller comprises a device suited to evaluate the condition of the spindles, configured so as to determine what spindles among the plurality of spindles are in the "present and valid yarn" state, and an average value calculator connected to the spindle condition evaluating device and configured so as to calculate the average value of the revolutions of the spindles in which the yarn is present and valid.

Owing to this embodiment, it is possible to calculate, possibly in real time, the frequency interval within which the yarn oscillation frequency can be considered acceptable.

In some embodiments, the controller comprises an interval calculator, configured so as to calculate an interval based on the average value of the revolutions and a threshold value, and wherein the interval calculator can be connected to the spindle condition evaluating device in such a way as to provide an interval value to the spindle condition evaluating device.

Owing to this embodiment, it is possible to evaluate whether the spindle is outside the calculated interval.

In some embodiments, the spindle condition evaluating device is configured so as to consider good the spindles having an oscillation frequency included within the interval.

Owing to this embodiment, it is possible to evaluate whether a spindle is in a condition that can be considered acceptable.

According to the invention, the controller comprises a chronometer and the spindle condition evaluating device is configured so as to consider not good the spindles having an oscillation frequency not included within the interval for a predetermined lapse of time measured by the chronometer and so as to be able to activate alarms.

Owing to this embodiment, it is possible to tolerate short variations in the yarn oscillation value outside the predetermined interval.

These variations may be due to production tolerances, small errors in the measurement of the oscillation frequency, or similar factors.

If these variations last for a short time, they can be filtered as measurement errors.

On the contrary, if the oscillation frequency remains outside the predetermined interval for a predetermined lapse of time, it will be possible to activate error systems.

### BRIEF LIST OF THE DRAWINGS

Further characteristics and advantages of the invention will be highlighted in greater detail through the analysis of the following description of some preferred but not exclusive embodiments, illustrated by way of indicative and not limiting example with the aid of the attached drawings.

In the drawings, the same reference numbers identify the same components.

In particular:
- Figure 1A shows a schematic sectional view of elements of a spinning frame 1000 known in the art. Figure 1B shows a schematic front view of elements of the spinning frame 1000;
- Figure 2A shows a schematic sectional view of elements of a spinning frame comprising a control device 2000. Figure 2B shows a schematic front view of elements of the spinning frame and of the control device 2000 of Figure 2A;
- Figure 2C shows an enlarged schematic sectional view of the control device 2000 of Figure 2A. Figure 2D shows an enlarged schematic front view of the control device 2000 of Figure 2B;
- Figure 3A shows a schematic sectional view of elements of a double yarn spinning frame comprising a control device 3000. Figure 3B shows an enlarged schematic front view of elements of the control device 3000 of Figure 3A;
- Figure 3C shows an enlarged schematic front view of the control device 3000 of Figure 3A used in the case of two yarns joined before passing in front of the device; Figure 3D schematically shows the position of one of the two yarns in the presence of a tear in the other yarn;
- Figure 4 shows an enlarged schematic front view of elements of a spinning frame comprising a control device 2000;
- Figure 5 schematically shows a control device 5000;
- Figure 6A schematically shows a frequency spectrum related to a yarn in a spinning frame; Figure 6B schematically shows part of the spectrum before a signal decimation treatment, while Figure 6C shows the result of the decimation process;
- Figure 7 schematically shows a control device 7000;
- Figure 8A schematically shows a signal frequency spectrum related to a yarn in a spinning frame; Figure 8B schematically shows an interpolation operation;
- Figure 9 schematically shows a control device 9000.

### DETAILED DESCRIPTION OF THE DRAWINGS

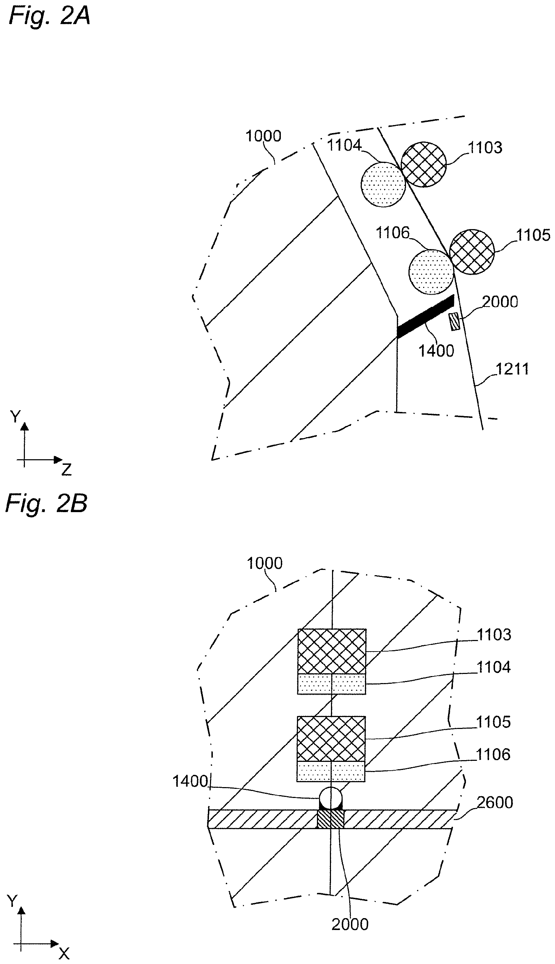

Figure 2A shows a schematic sectional view of elements of a spinning frame comprising a control device 2000. Figure 2B shows a schematic front view of elements of the spinning frame and of the control device 2000 of Figure 2A.

The spinning frame can substantially correspond to the spinning frame 1000 with the omission of the sensor 1300, whose function is replaced and improved by the control device 2000. More generally, however, the control device 2000 can be installed on several types of spinning frame, even different from the one illustrated herein.

The control device 2000 can be positioned in proximity to the last pair of drawing rollers 1105-1106, possibly under the suction pipe 1400, or in some cases over it, when the latter is provided. In some embodiments, the control device 2000 can be installed at a distance of approximately 10 mm under the suction pipe 1400 and/or of approximately 30 mm under the last pair of rollers 1105-1106. More generally, it is sufficient that the device 2000 is positioned in proximity to the yarn 1211, in such a way that it is able to detect its presence or absence, and possibly its movement, as described in greater detail below. It will thus be possible to install the device 2000 even in a lower position along the direction Y, for example just below the yarn guide lappet 1108.

The control device 2000 can be installed on a supporting bar 2600, for example an aluminium raceway, which provides mechanical strength to the system without creating problems to other elements of the spinning frame. In some embodiments described by way of example, the supporting bar can be approximately 20 mm in height and 15 mm in depth. Furthermore, this makes it possible to prevent the device 2000 from being positioned on the ring rail 1215, avoiding the problems described above. The supporting bar can be fixed to the body 1107 of the spinning frame, thus also advantageously avoiding to increase the external dimensions of the spinning frame 1000, in particular in the case where, as illustrated, the device 2000 is placed between the yarn 1211 and the body 1107. Finally, the presence of the supporting bar 2600 makes it possible to install in a simple manner a plurality of devices 2000 for a plurality of spindles. In addition to the above, the supporting bar 2600 allows any cables for connection to one or more devices 2000 to be housed inside it.

It will thus be clear that such a configuration, furthermore, advantageously makes it possible to adapt existing spinning frames 1000 using the control device 2000 or a plurality of control devices 2000 installed on the supporting bar 2600. In addition or as an alternative to the above, in some embodiments it will be possible to connect the supporting bar 2600 to several parts of the spinning frame, in order to provide a higher degree of stability to the supporting bar 2600. Furthermore, the position of the device 2000 in proximity to the yarn 1211 makes it possible to avoid the accumulation of fibres on the sensors of the device 2000, due to the small air vortices generated by the movement, and oscillations of the yarn 1211. As an alternative or in addition to the above, installation in proximity to the suction pipe 1400 allows any fibres or dirt deposited on the device 2000 to be sucked by the suction pipe, with evident advantages in terms of reduced maintenance and increased reliability.

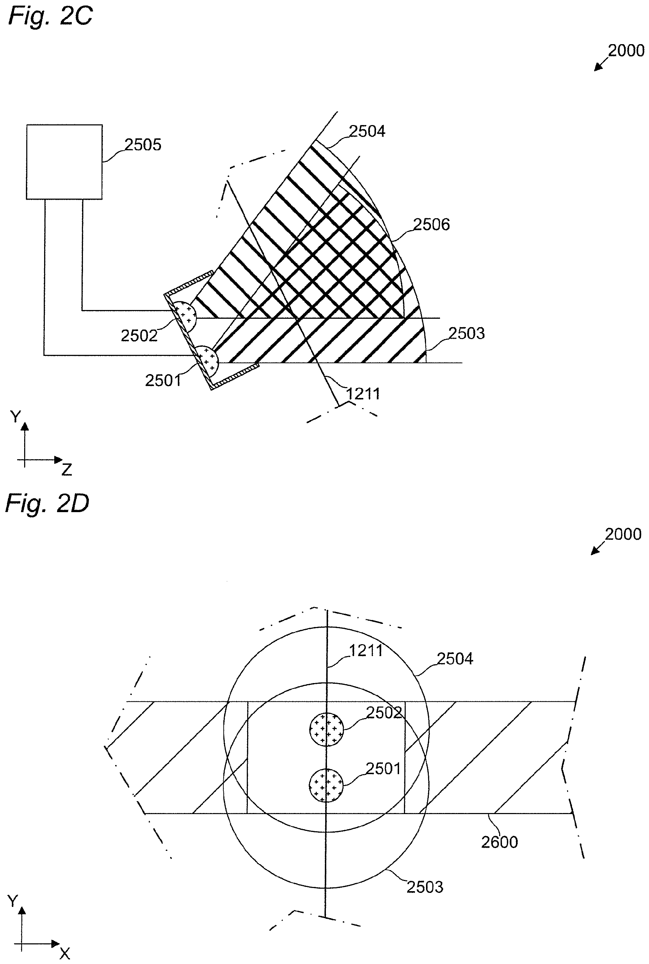

Figure 2C shows an enlarged schematic sectional view of the control device 2000 illustrated in Figure 2A. Figure 2D shows an enlarged schematic front view of the control device 2000 illustrated in Figure 2B.

As can be seen, the control device 2000 comprises an optical generator 2501, an optical detector 2502 and a controller 2505.

The optical generator 2501 can be, for example, an infrared photodiode or any element capable of generating a radiation having a predetermined emission value in a first radiation emission region 2503. The first radiation emission region 2503 can be in a substantially conical shape, as illustrated, but the invention is not limited to this implementation. Any shape of the first radiation emission region 2503 that allows the yarn 1211 to be irradiated during normal operation of the spinning frame can be implemented.

In an analogous manner, the optical detector 2502 can be a light sensitive diode or any element capable of transforming a brightness measure into an electrical measure. The optical detector 2502 measures irradiation by reflection in a first radiation detection region 2504. The first radiation detection region 2504 can be in a substantially conical shape, as illustrated, but the invention is not limited to this implementation. Any shape of the first radiation detection region 2504 that allows a radiation originating from the region where the yarn 1211 is situated to be measured during normal operation of the spinning frame can be implemented. The yarn 1211, in its normal operating position, that is, in the absence of tears, is subjected to the radiation emitted by the optical generator 2501. Part of this radiation is reflected by the yarn and measured by the optical detector 2502.

As can be seen in Figure 2C, there is no need for the radiation emission region 2503 and the radiation detection region 2504 to occupy spatially corresponding positions. In some embodiments, it is sufficient that at least a part of each region creates an overlapping region 2506. Even more generally, it will be sufficient that the reflection produced by the yarn 1211 is at least partially included in the radiation detection region 2504. The optical generator 2501 thus irradiates the yarn 1211 that reflects a part of the radiation measured by the optical detector 2502. The output signal emitted by the optical detector 2502 is sent to the controller 2505, which can thus determine, for example, the absence of the yarn 1211, if the value of irradiation by reflection is lower than a predetermined value. Furthermore, when the yarn moves with respect to the optical detector 2502, in the direction X and/or in the direction Z, the value measured by the optical detector 2502 changes, according to the movement of the yarn. In this way, as described below, it is possible to detect not only the presence the yarn, but also its movement and the corresponding frequency of movement.

As illustrated, it is also possible to connect the optical generator 2501 to the controller 2505. The controller 2505 makes it possible to adjust the predetermined emission value of the radiation generated by the optical generator 2501, possibly based on the information received from the optical detector 2502. This solution is advantageous, as it makes it possible to adjust the sensitivity of the device 2000, as described below.

For example, if the signal received from the optical detector 2502 is lower than a predetermined threshold value, it will be possible to increase the emission power of the optical generator 2501.

The controller 2505 can be placed anywhere in the spinning frame 1000, making the device 2000 very compact and flexible. In general, it is preferable to position the controller 2505 in a portion of the body 1107 free from other elements. Alternatively, it will be possible to place the controller 2505 on the supporting bar 2600.

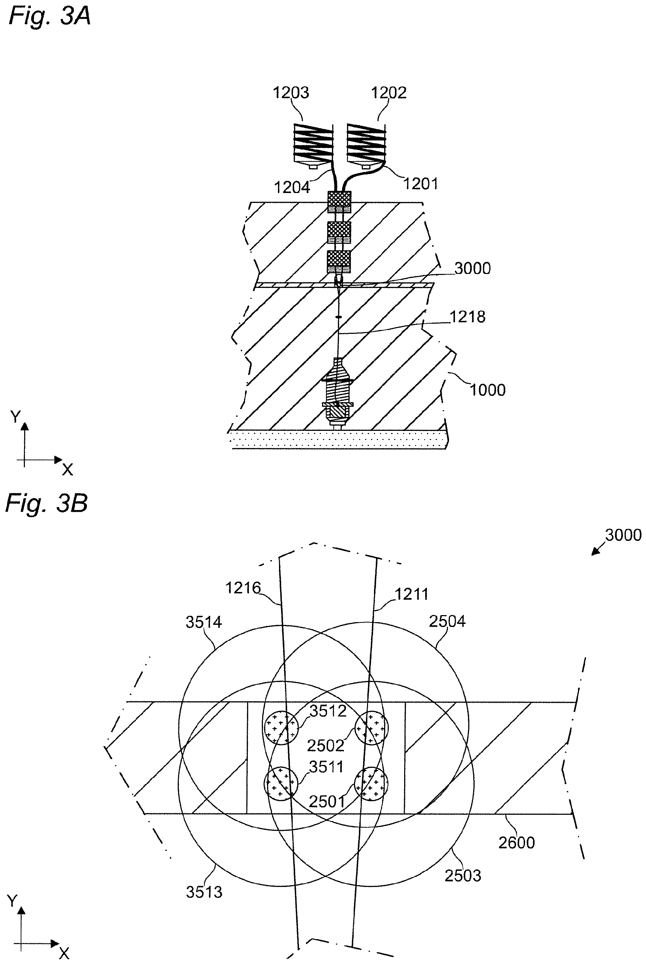

Figures 3A and 3B show a view of a control device 3000, in the case where the two yarns 1211 and 1216 are twisted together. The two yarns may be similar to each other or even have different characteristics, such as colour, thickness or materials.

In this embodiment, using two optical generators 2501 and 3511 and two optical detectors 2502 and 3512 it is possible to create two radiation emission regions 2503 and 3513 and two radiation detection regions 2504 and 3514. Making each yarn 1211 and 1216 pass substantially inside a single pair made up of radiation emission region and radiation detection region, it is possible to detect the movement of the single yarn 1211, 1216 by means of the respective optical detector.

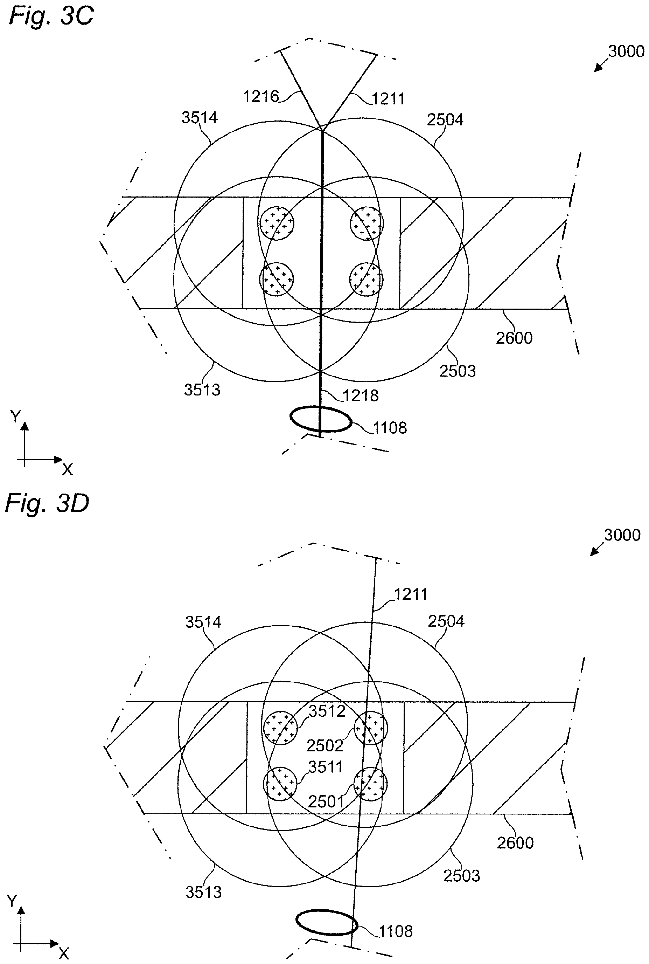

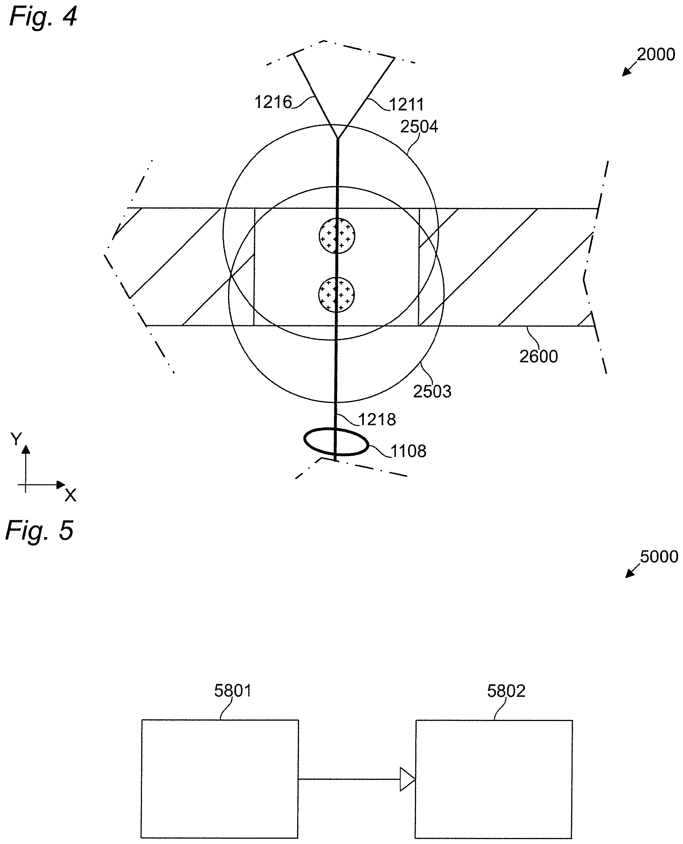

Alternatively, it will be possible to position just one control device 2000 in the region where the two yarns 1211 and 1216 are already joined, as shown in Figure 4. This form of implementation ensures more flexibility in the positioning of the control device along the yarn resulting from the combination 1218 of yarns 1211 and 1216. More generally, both the control device 2000 and the control device 3000 can be arranged in any position in which the yarn is allowed to pass within at least one radiation emission region and one radiation detection region 2504. Furthermore, as shown in Figures 3C and 3D, it is possible to use the control device 3000 also in the case of two yarns joined before passing in front of the device. As can be seen, in fact, in case of breakage of one of the two yarns, in the example the yarn 1216, the remaining yarn 1211 will move to a position which is determined by the position of the last pair of rollers 1105, 1106 and the yarn guide lappet 1108.

In case of use of the control device 3000 with two optical pairs, one with the generator 2501 and the detector 2502, and the other with the generator 3511 and the detector 3512, to detect the movement of the yarn and the corresponding movement frequency it will be possible to use the pair having the output signals emitted by the detector 2502 or 3512 with the best characteristics, as described here below.

The forms of implementation described up to now generally make it possible to determine whether one or more yarns are present or not, in addition to detecting the movement of the same. In particular, it is possible to use the output signal emitted by the detectors 2502, 3512, or a corresponding signal processed by the controller 2505 in order to measure whether the twisting action exerted on the yarns 1211, 1216 and/or on their combination 1218 is correct or not. In general, this measure can be obtained by measuring the vibration frequency of the yarn, obtained from the signal emitted by the detector 2502 and/or 3512 or the respective signal processed by the controller 2505. It is clear that here below any reference to the yarn may be intended to indicate, for the sake of brevity and clarity, the individual yarn 1211, the individual yarn 1216, or their combination 1218.

In greater detail, the yarn moves with respect to the detector due to the rotation transmitted by the ring traveller 1213. The rotation speed of the ring traveller 1213 indicates the correct twisting of the yarn. The vibration transmitted to the yarn, in fact, is directly linked to the rotation speed of the ring traveller 1213. It is thus possible to measure the vibration frequency of the yarn and to determine, starting from this value, whether the rotation speed of the ring traveller 1213 falls within a range of values that is considered acceptable.

The vibration frequency of the yarn can be determined because the movement of the yarn with respect to the optical detector 2502, 3512 varies the amplitude of the signal measured by the latter. Thus, analysing the output signal emitted by the optical detector 2502, 3512, or by the controller 2505, it is possible to determine the vibration frequency of the yarn. In particular, the output signal emitted by the detector 2502, 3512, or by the controller 2505 is an amplitude-modulated signal, whose modulating wave has components in frequency linked to the revolutions with which the yarn is wound on the spool 1212, as explained below.

As shown in Figure 5, a control device 5000 comprises an optical movement sensor 5801 and a frequency meter 5802 connected to the optical movement sensor 5801. Even if they are represented separately, in some specific embodiments the optical movement sensor 5801 and the frequency meter 5802 can be physically integrated or be made on a single PCB.

The optical movement sensor 5801 can, for example, comprise the detector 2502 and/or 3512. In addition to the above, it may also comprise the generator 2501 and/or 3511, and/or the controller 2505. Alternatively, the optical movement sensor 5801 may comprise the control device 2000 or 3000. More generally, the optical movement sensor 5801 can be any device that makes it possible to detect in an optical manner the movement of the yarn, preferably along a plane that is substantially perpendicular to the sliding plane of the yarn.

The optical movement sensor 5801 therefore is configured in such a way as to measure the movement of the yarn. The frequency meter 5802 is configured so as to receive the value measured by the optical movement sensor 5801 and so as to measure at least one yarn oscillation frequency.

In general, if the vibration frequency does not fall within a predetermined range of values, it is possible to conclude that the rotation of the yarn around the bobbin 1212 presents a malfunction. In this way, it is possible to determine whether the rotation of the yarn is taking place correctly and the yarn is being twisted correctly, too. This in turn indicates that the spinning process is being performed correctly.

In a generic form of implementation of the invention, it will thus be possible to measure the vibration frequency of the yarn. If this frequency does not fall within a predetermined interval, it will be possible to conclude that there is a problem in the spindle and to activate error management procedures, for example, to lock the spindle, and/or to emit an optical and/or acoustic signal, and/or to activate a roving locking device, etc.

In a specific form of implementation of the present invention, the optical movement sensor 5801 may comprise the optical generator 2501, 3511, or a similar optical generator, configured so as to emit a modulated radiation, for example a square wave, sinusoidal wave or sawtooth wave radiation with a predetermined frequency F1, for example in the interval 1kHz-10kHz.

If the yarn is present, there will be a part of the radiation that is reflected and returns towards the detector 2502, 3512. The wave generated by the reflection will have a carrier with frequency corresponding to the frequency F1 of the radiation generated by the generator 2501, 3511 and a modulating wave corresponding to the vibration of the yarn, possibly with different components in frequency.

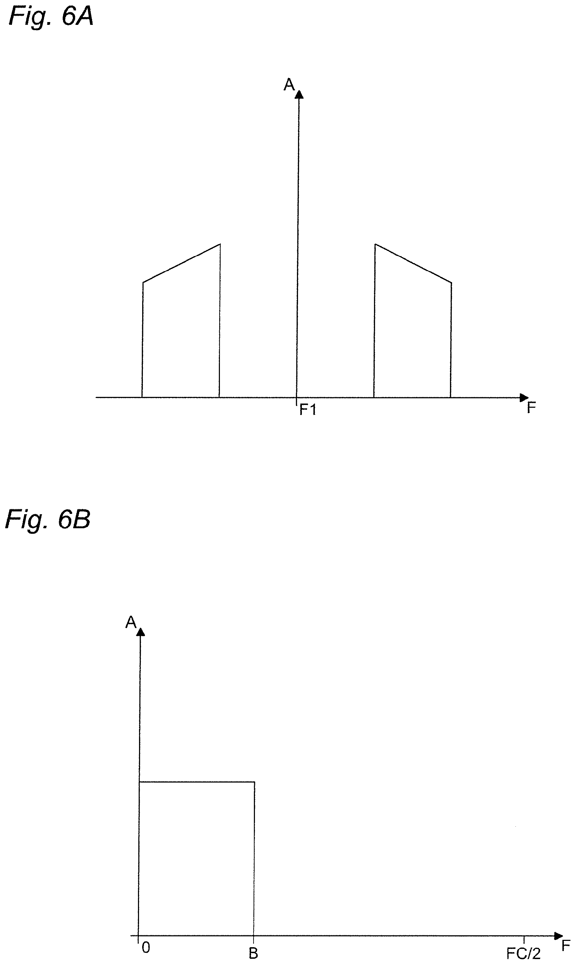

An example of spectrum of the signal measured by the detector 2502, 3512 is illustrated in Figure 6A. The spectrum is that of a typical amplitude modulation (AM) in which the carrier, corresponding to the predetermined frequency F1 of the radiation generated by the generator, is centred in the spectrum and the vibration of the yarn creates two frequency lobes that are symmetrical with respect to the carrier F1.

As the vibration of the yarn is mainly caused by its winding on the spool 1212, substantially round, by means of the ring traveller 1213 that rotates around the circular ring 1214, in first approximation it is possible to consider that the vibration transmitted by the rotation will be a sinusoidal wave, with frequency corresponding to the rotation speed of the yarn around the spool 1212. A noise resulting from other movements of the spinning frame, for example the movement of the ring rail 1215, from an irregularity in the roving 1201, 1204 and/or the yarn, mechanical vibrations, etc. can be added to this frequency. Thus, analysing the spectrum of the signal measured by the optical detector 2502, 3512 it is possible to determine the transmission carrier and, in the specular area of the modulating wave AM, a predominant harmonic corresponding to the main vibration of the yarn which is caused by its rotation around the spool 1212. If the value of the predominant harmonic does not fall within a predefined interval, as described below, it can be concluded that there is a problem in this spindle and the error management procedures described above can be activated.

The methods for determining the frequency corresponding to the rotation of the ring traveller 1213 around the spool 1212 are multiple and will be clear for the experts in the art.

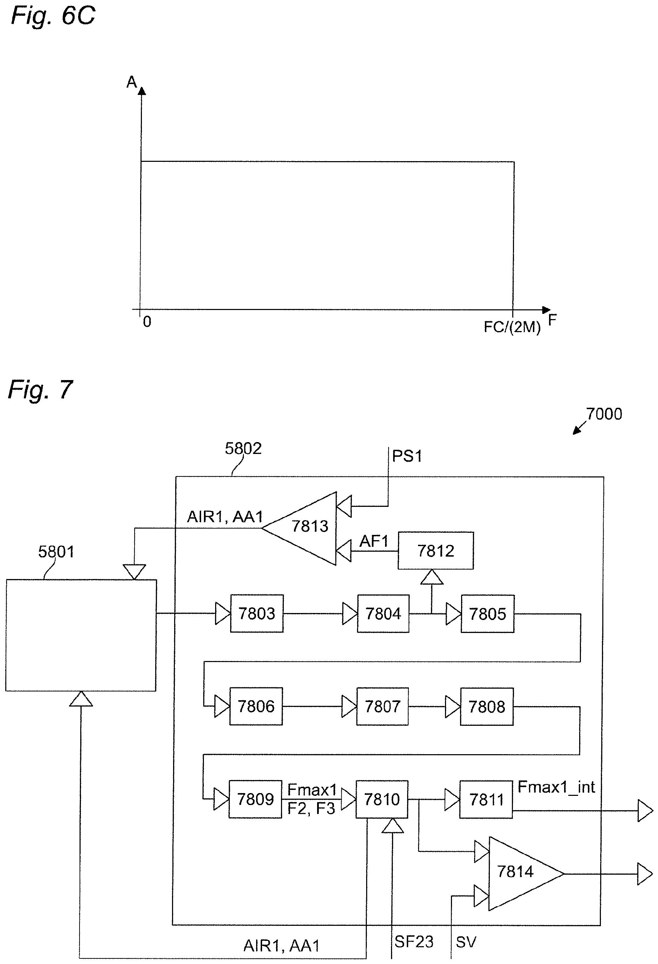

A specific implementation of a control device 7000 is described here below by way of non-limiting example, with reference to Figure 7. In particular, the control device 7000 comprises the optical movement sensor 5801 of the control device 5000 and a series of components that create a possible specific implementation of the frequency meter 5802.

As can be seen in Figure 7, the output signal emitted by the optical movement sensor 5801 is filtered by a low-pass filter 7803, preferably discrete, and sampled by a sampler 7804. The sampling frequency respects the Nyquist-Shannon theorem. For example, considering as maximum frequency value 30,000 revolutions per minute, that is, 500 Hertz, and a 2kHz carrier F1, it will be possible to sample at 5kHz or above.

A band-pass filter 7805 is applied to the samples leaving the sampler 7804, in such a way as to filter only one modulation component, for example the right lobe illustrated in Figure 6A, and the carrier F1 of the signal. In a specific form of implementation it will be possible to select a band included between F1 and F1+500 Hertz, or more generally F1 added to the maximum rotation frequency of the spool 1212.

The samples, filtered by the band-pass filter 7805, are translated in frequency by a translator 7806, for example a digital mixer, in such a way as to move the carrier of the signal from the value F1 to a frequency of 0Hz or to a substantially similar value. Successively, the samples are filtered again by a low-pass filter 7807, preferably digital, in such a way as to eliminate possible aliasing phenomena that may occur during the frequency translation and that may occur in the successive decimation step.

The samples are successively subjected to a decimation operation by a decimator 7808, for example by a factor M of a power of two, in such a way as to zoom on a pre-fixed frequency interval. The result of this operation is illustrated in Figures 6B and 6C.

In particular, Figure 6B schematically shows part of the spectrum before the signal decimation treatment, while Figure 6C shows the result of the decimation process.

Specifically, as can be seen in Figure 6B, the input signal arriving at the decimator 7808 has a small band B, if compared to the potential field of analysis included between zero and FC/2, where FC is the sampling frequency. In order to obtain a more precise analysis of the signal, the decimator 7808 reduces the sampling frequency FC by a factor M.

Successively, a transformer 7809, for example a 1024 or 512 point transformer of the Fast Fourier Transform type, is applied to the samples after the decimation operation in order to allow the received signal to be analysed. Indicatively, for example, with a sampling frequency FC equal to 5kHz, a factor M equal to 2 and 1024 points, a frequency resolution of approximately 2.44Hz is obtained, corresponding to approximately 146 RPM.

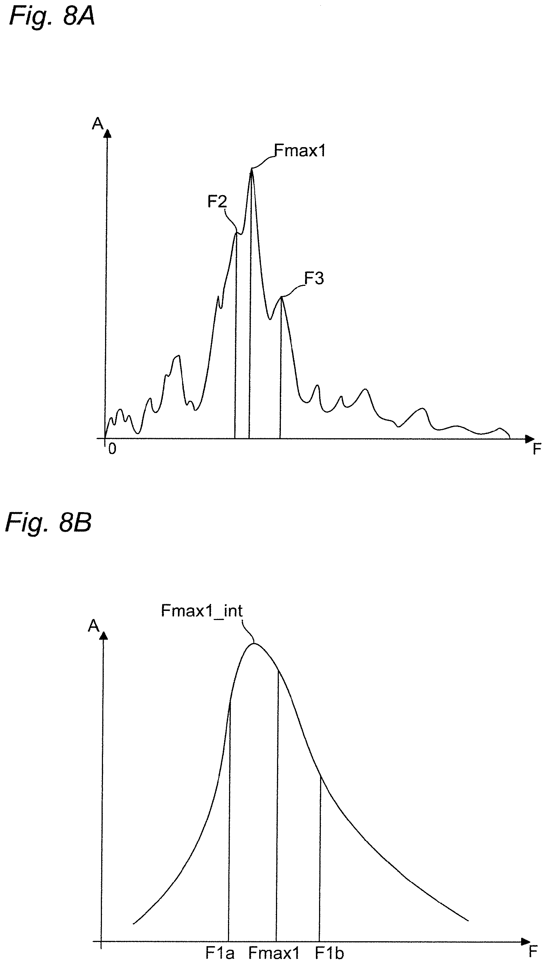

The previous steps thus result in a signal indicating the yarn vibration spectrum, as schematically illustrated, for example, in Figure 8A. In particular, the figure schematically shows the signal in amplitude A with respect to the frequency F, obtained from the previous steps.

In order to determine the main vibration frequency of the yarn, that is, as described above, the frequency depending on the rotation of the same around the spool 1212, it is sufficient to determine the harmonic Fmax1 with the greatest amplitude, possibly using known techniques. In some embodiments, the value Fmax1 determined in this way can then be used directly as an indicative value of the yarn vibration frequency, and thus be used to determine, as described above, whether each individual spindle is working correctly or not.

In some embodiments, it will furthermore be possible to apply an optional interpolation algorithm, schematically represented by the interpolator 7811, in such a way as to make it possible to find a more precise value of the modulating frequency called Fmax1_int. A schematic view illustrating the operation of the interpolator 7811 is shown in Figure 8B, where Fmax1_int indicates the result of the interpolator based on the frequencies Fmax1 and on its two nearest harmonic frequencies F1a and F1b. In other words, the value Fmax1_int is determined as the frequency having the greatest amplitude in the signal obtained from the interpolation passing through point Fmax1 and at least through the two harmonics F1a and F1b that are nearest to Fmax1. It will be clear that the number of harmonic frequencies can be higher than two, and the distribution of the same is not necessarily symmetrical with respect to the value of Fmax1. Thus, the frequency Fmax1_int corresponds to an interpolated value of the rotation of the yarn around the spool 1212. For example, with reference to Figures 8A and 8B, in the case where the frequency Fmax1_int is 90Hz, the rotation value will be 90Hz x 60 revolutions per minute, that is, 5.400 revolutions per minute.

In some forms of implementation, the control device 7000 can thus comprise also an optional spurious frequency detector 7810.

In particular, it is possible that, as can be seen in Figure 8A, in addition to the frequency Fmax1 there are spurious harmonics, for example F2 and F3, whose amplitude is smaller but still comparable to the frequency Fmax1. These can be due, for example, to excessive radiation reflected by the yarn, which saturates the amplification and filtering electronic circuits, or more generally to distortions of the input signal. These distortions can make it more difficult to carry out a precise measurement of the frequency Fmax1.

In this case, the spurious frequency detector 7810 detects the spurious harmonics, for example F2 and F3, and compares their amplitude value with that of the frequency Fmax1. The spurious frequency detector 7810 can be connected to the optical movement sensor 5801 and/or to the controller 2505 in such a way as to modify the power of the radiation generated by the optical generator 2501, 3511 and/or the amplification value of the value measured by the optical movement sensor 5801, as described below.

In particular, in the spurious frequency detector 7810, the amplitude of Fmax1 is compared to the amplitudes of any spurious frequencies F2, F3. The amplitudes of F2, F3 are compared, through a comparator not illustrated herein, to a threshold SF23 that can be set by the user and is defined as a percentage of the amplitude of Fmax1. The value of SF23 can be included, for example, between 0 and 90%, preferably between 0 and 75%, even more preferably between 0 and 50%.

If the amplitudes of the spurious frequencies F2, F3 are lower than the value of Fmax1 multiplied by the threshold value SF23, they can be considered as spurious frequencies and therefore eliminated from the analysis of the interpolator 7811.

On the contrary, if the amplitudes F2, F3 are higher than the value of the amplitude of Fmax1 multiplied by the threshold value SF23, the spurious frequency detector 7810 can emit an output control on signal AIR1 and/or on signal AA1. In particular, the signal AIR1 represents the irradiation power of the optical generator 2501, 3501, while the signal AA1 represents the amplification gain of the optical detector 2502, 3512, and/or of the controller 2505. More particularly, one or both of these signals can be reduced. If they are both reduced, the reduction can be simultaneous or it will be possible to reduce first one signal and then the other. The reduction can be carried out with calibration steps. The user will possibly be able to set the calibration steps, to select the signal to be reduced and even to decide whether the signals must be reduced simultaneously or one after the other.

Through the control of AIR1 and AA1, it is thus possible to obtain a focusing action through calibration steps, in order to obtain an amplitude of Fmax1 sufficiently different from the amplitude of the spurious frequencies F2, F3.

In some forms of implementation, the control device 7000 can also comprise an effective value meter 7812.

In particular, the effective value meter 7812 can measure the effective value (RMS) of the samples leaving the sampler 7804. The effective value measured in this way, called AF1, can be used to analyse the presence of the yarn, as described below. For example, if 100 is assumed as maximum value of AF1, said maximum value could practically correspond to the saturation value of the output signal of the module 7812. Said value AF1 is compared, by means of a comparator 7813, which is optional, too, to a value PS1 which can possibly be set by the user. The value PS1 represents a presence threshold, which for example can be set as 50% of AF1, preferably 70%, even more preferably 80%.

If AF1 exceeds or is equal to PS1, the yarn is considered as present.

If, on the contrary, AF1 is lower than PS1, it is possible to increase the irradiation power AIR1 of the optical generator 2501, 3511 and/or the amplification AA1 of the value measured by the optical movement sensor 5801, possibly up to the maximum settable value, until the value AF1 exceeds the value PS1. For the reduction of the values of AIR1 and/or AA1, that which has been described above applies.

It is clear that it is also possible to determine the presence and the absence of the yarn in different manners, for example by means of a simple reflection optical device or mechanical feelers. In this case, the modules 7812 and 7813 may be replaced by the output of said devices. In general, the presence of the modules 7812 and 7813 is however optional, since in any case it is possible to proceed with the analysis of the frequency, even without measuring the effective value. The setting of the irradiation power AIR1 and/or of the gain of the amplification AA1 of the measured value can thus be carried out, in some forms of implementation, in a concurrent manner by the modules 7813 and 7810. In some forms of implementation, if the modules 7810 and 7813 cannot adjust the value of AIR1 and/or AA1 in such a way as to exceed both of the thresholds PS1 and SF23, it can be concluded that there is a problem in the spindle and the error management procedures described above can be activated.

The continuous control of AIR1 and/or AA1 makes it possible to follow the variation in the distance of the yarn from the detector 2502, 3512, which may occur due to the movement of the ring rail 1215. As an alternative or in addition to the above, these adjustments can furthermore be used to compensate for the variation in the thickness, even of a single yarn, and/or in the yarn colour, which cause different variations in the reflected radiation, and also to compensate for any deposits of fibres on the surface of the sensor.

In optional forms of implementation, if it is not possible to adjust the irradiation power AIR1 and/or the amplification AA1 in such a way as to obtain a value of the amplitude of Fmax1 substantially different from the amplitude of the spurious harmonics F2 and F3 and/or in such a way as to obtain a value of AF1 higher than PS1, the control device 7000 can use a second channel, if available. In particular, in the case of use of two optical generators 2501 and 3511 and two optical detectors 2502, 3512, it will be possible to use first one generator-detector pair and successively a second generator-detector pair.

Alternatively, it will be possible to evaluate the output signal of both pairs and to select the signal that offers the best measurement conditions.

In some optional embodiments, it will furthermore be possible to measure the value of the amplitude of Fmax1 and proceed with the analysis by means of the interpolator 7811 only if the amplitude of Fmax1 exceeds a predetermined threshold.

In particular, the ampitude of Fmax1 can be compared to a threshold value SV, by means of an optional comparator 7814. This threshold SV can be adjusted and set, for example on a scale 0-100, wherein 100 is the maximum possible amplitude of the received signal Fmax1. If the amplitude of Fmax1 is lower than SV, the current vibration is not considered valid. This can, for example, be the case when a broken yarn rests on the sensor and therefore results to be stationary, with no vibrations, and therefore broken. If the amplitude of Fmax1 exceeds SV, the process continues with the analysis of Fmax1. It is clear that the comparator 7814 can be connected both before and after the spurious frequency detector 7810.

By means of the control devices 5000 and 7000 it is possible to determine the presence or absence of the yarn, as described above. It is furthermore possible to determine the yarn oscillation frequency, Fmax1_int, which is representative of the rotation of the yarn around the spool 1212. The signal Fmax1_int can be used directly by the control device to determine the presence of an error on the spindle on which the control device is installed, if the frequency is not included between a predetermined interval that can possibly be defined by the user.

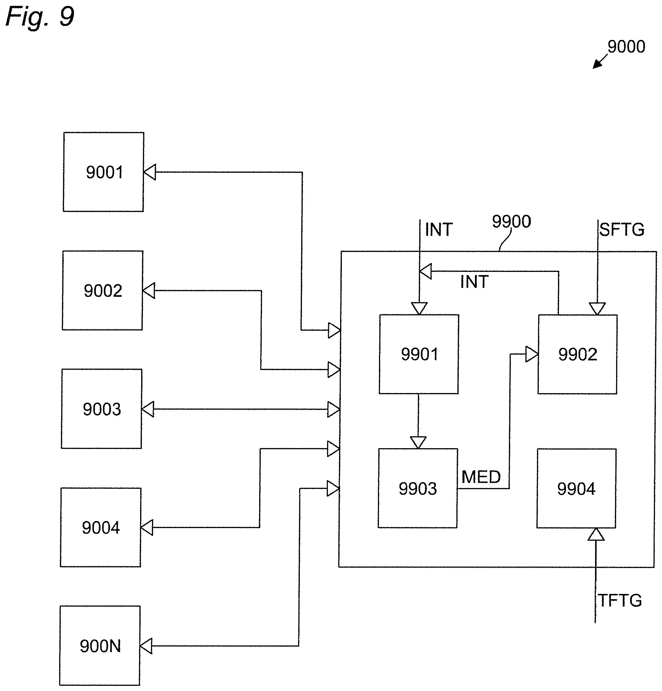

In some forms of implementation it is also possible to collect and gather the information provided by several individual control devices, in particular the various oscillation frequencies of the various yarns, and to use this information to define the predetermined interval within which the oscillation frequency can be considered good. In particular, a control system 9000 allowing this type of use is described below with reference to Figure 9.

As can be seen in Figure 9, the control system 9000 comprises a plurality of control devices 9001-900N, which if necessary can be grouped in a raceway. Each one of the control devices 9001-900N can be either of the control devices 5000 or 7000 previously described above. The control devices 9001-900N can represent the entirety of the control devices mounted on the spinning frame or just a group of them.

The control system 9000 comprises also a controller 9900 connected to each one of the control devices 9001-900N. The controller 9900 receives the information indicating the presence/absence or breakage of the yarn from one or more of the control devices 9001-900N, for example through the output signal of the comparator 7813 and/or the comparator 7814. In addition or as an alternative to the above, the controller 9900 receives the information indicating the vibration frequency of the yarn from one or more of the control devices 9001-900N, for example through the signal Fmax1, or Fmax1_int.

The controller 9900 is configured in such a way as to evaluate which of the control devices is in a condition defined as good. This is implemented through a yarn condition evaluating device 9901 configured so as to determine what spindles among the plurality of spindles are in a good condition. The determination of the good condition can take place by evaluating as good the spindles whose yarn oscillation frequency is included within an interval INT which can be predetermined or can be set by the user or can be determined by the controller 9900, as described below.

Based on the spindles whose signal indicates that the yarn is present and not broken, meaning spindles with an acceptable oscillation frequency, the controller 9900 can calculate the average number of revolutions MED, using the information related to the oscillation frequency received from the control devices 9001-900N, through an average value calculator 9903. In particular, the average value calculator 9903 receives the information indicating what spindles have a signal indicating that the yarn is present and not broken through a connection to the yarn condition evaluating device 9901. In some forms of implementation, all the spindles can be initially considered good on setting in operation of the control system 9000.

A value SFTG, which can be predetermined or can be set by the user, defines the threshold percentage to be considered to determine what spindles have a number of revolutions outside the tolerance interval. For example, SFTG may have a value included between 0 and 50%, more preferably between 0 and 25%, even more preferably between 0 and 2%. An interval calculator 9902 calculates the interval INT of number of revolutions within which the spindle is considered good, based on the values of MED and SFTG, for example calculating INT as included between MED^{∗}(1-SFTG) and MED^{∗}(1+SFTG).

Based on the same evaluation, the controller 9900 can send a signal to each one of the control devices 9001-900N to indicate that the spindle is outside the tolerance interval, if the frequency measured by the respective control device 9001-900N is not included in the interval INT. Based on this information, the respective control device 9001-900N can activate the error management procedures described above. Alternatively or in addition to the above, the control of the different error management procedures for the different spindles can be centralized and performed by the controller 9900. Again alternatively or in addition to the above, it will be possible to notify the respective control device 9001-900N, which can immediately activate the error management procedures, though offering also information centralized by the system 9000, via a screen not illustrated herein or similar components.

According to the invention, the controller 9900 comprises a chronometer 9904. A value TFTG, which can be predetermined or can be set by the user, defines a duration, for example of 10 seconds, preferably 5 seconds, even more preferably 1 second. The controller, through the chronometer 9904, is configured so as to evaluate, for each control device 9001-900N, whether the spindle associated with the respective control device 9001-900N is outside the interval INT for a time exceeding the value TFTG. In this form of implementation, only if the frequency value indicated by the control device 9001-900N remains outside the interval INT for a time equal to or longer than TFTG, the controller will consider the spindle not good, thus excluding it from the calculation of the average value MED and notifying the control device 9001-900N of the fact that the spindle is outside the tolerance interval.

In some forms of implementation, the controller 9900 can alternatively obtain the average rotation value MED through a sensor, not illustrated, positioned on the motor that sets the spindles rotating. Or the value MED can be set by the user. Even if some embodiments have been described independently, it is clear that they can be combined and that the invention is defined by the claims and is not limited to the individual embodiments described herein.

## Claims

1. Control system (9000) for spinning frames (1000), the spinning frames (1000) comprising a plurality of spindles, each spindle comprising first drawing rollers (1101-1106) configured so as to draw a first roving (1201) and thus obtain a first yarn (1211, 1216, 1218),
the control system (9000) comprising a plurality of control devices (5000, 7000, 9001-900N) associable to the plurality of spindles, each control device (5000, 7000, 9001-900N) comprising:
- an optical movement sensor (5801), and
- a frequency meter (5802),
**wherein** the optical movement sensor (5801) is configured so as to measure the movement of the first yarn (1211, 1216, 1218),
**wherein** the frequency meter (5802) is configured so as to receive the signal measured by the optical movement sensor (5801) and so as to measure at least one oscillation frequency (Fmax1_int) of the first yarn (1211, 1216, 1218),
**characterized in that**
the control system (9000) further comprising a controller (9900), **wherein** the controller comprises:
- a device for evaluating the condition of the spindles (9901), configured so as to determine at what spindles of the plurality of spindles the first yarn is present and in a valid yarn state, and
- an average value calculator (9903) connected to the device for evaluating the condition of the spindles (9901), configured so as to calculate an average value of the revolutions (MED) of the spindles at which the first yarn is present and in a valid yarn state, or
- a sensor, configured to be positioned on a motor which rotates the spindles, configured so as to measure the average value of revolutions (MED) as the value of revolutions of the motor, and
- an interval calculator (9902) configured so as to calculate an interval (INT) based on the average value of revolutions (MED) and on a threshold value (SFTG),
**wherein** the interval calculator (9902) is connected to the device for evaluating the condition of the spindles (9901) so as to provide the value of the interval (INT) to the device for evaluating the condition of the spindles (9901), **wherein** the controller (9900) is connected to the plurality of control devices (5000, 7000, 9001-900N) so as to receive the oscillation frequency (Fmax1_int) measured by the respective control device (5000, 7000, 9001-900N),
and **wherein** the device for evaluating the condition of the spindles (9901) is configured so as to evaluate as good the spindles whose oscillation frequency is included within the interval (INT),
**wherein** the controller (9900) comprises a chronometer (9904), and **wherein** the device for evaluating the condition of the spindles (9901) is configured so as to evaluate as not good the spindles whose oscillation frequency is outside the interval (INT) for a predetermined time duration (TFTG) measured by the chronometer (9904) and so as to be able to activate an alarm.

2. Control system according to claim 1, **wherein** the optical movement sensor (5801) comprises an optical generator (2501, 3511) configured so as to emit a modulated radiation with a predetermined frequency (F1).

3. Control system according to claim 2, **wherein** the frequency meter (5802) comprises a spurious frequency detector (7810) configured so as to determine the presence of at least one spurious frequency (F2, F3) in the signal measured by the optical movement sensor (5801).

4. Control system according to claim 3, **wherein** the spurious frequency detector (7810) is configured so as to reduce and/or increase the power (AIR1) of the modulated radiation and/or the amplification (AA1) of the measured signal if the presence of at least one spurious frequency (F2, F3) is determined.

5. Control system according to any of the preceding claims, furthermore comprising a controller (2505) connected to the frequency meter (5802), **wherein** the controller (2505) is configured so as to determine the presence of a problem if the oscillation frequency (Fmax1_int) is outside the predetermined interval (INT).

## Patentansprüche

1. Steuerungssystem (9000) für Spinnmaschinen (1000), wobei die Spinnmaschinen (1000) eine Vielzahl von Spindeln umfassen, wobei jede Spindel erste Zugwalzen (1101-1106) umfasst, die so konfiguriert sind, dass sie ein erstes Vorgarn (1201) ziehen und dann ein erstes Garn t(1211, 1216, 1218) gewinnen,
wobei das Steuerungssystem (9000) eine Vielzahl von Steuergeräten (5000, 7000, 9001-900N) umfasst, die der Vielzahl von Spindeln zugeordnet werden können, wobei jedes Steuergerät (5000, 7000, 9001-900N) Folgendes umfasst:
- einen optischen Bewegungssensor (5801), und
- einen Frequenzmesser (5802),
wobei der optische Bewegungssensor (5801) so konfiguriert ist, dass er die Bewegung des ersten Garns (1211, 1216,1218) misst,
wobei der Frequenzmesser (5802) so konfiguriert ist, dass er das durch den optischen Bewegungssensor (5801) gemessene Signal empfängt und so, dass er wenigstens eine Schwingungsfrequenz (Fmax1_int) des ersten Garns (1211,1216,1218) misst,
**dadurch gekennzeichnet, dass**
das Steuerungssystem (9000) des Weiteren einen Regler (9900) umfasst, **wobei** der Regler Folgendes umfasst:
- eine Vorrichtung zur Auswertung des Zustands der Spindeln (9901), so konfiguriert, dass er bestimmt, an welchen Spindeln der Vielzahl von Spindeln das erste Garn vorhanden und in einem gültigen Garnzustand ist, und
- einen an die Vorrichtung zur Auswertung des Zustands der Spindeln (9901) angeschlossenen Mittelwertrechner (9903), so konfiguriert, dass er einen Umdrehungs-Mittelwert (MED) der Spindeln berechnet, an denen das erste Garn vorhanden und in einem gültigen Garnzustand ist, oder
- einen Sensor, dazu konfiguriert, an einem Motor für die Spindeldrehung positioniert zu werden, und so konfiguriert, dass er den Umdrehungs-Mittelwert (MED) als Wert der Motorumdrehungen misst, und
- einen Intervallrechner (9902), dazu konfiguriert, ein auf dem Umdrehungs-Mittelwert (MED) und auf einem Schwellenwert (SFTG) basiertes Intervall (INT) zu berechnen,
**wobei** der Intervallrechner (9902) an die Vorrichtung zur Auswertung des Zustands der Spindeln (9901) angeschlossen ist, um den Wert des Intervalls (INT) an die Vorrichtung zur Auswertung des Zustands der Spindeln (9901) zu liefern,
**wobei** der Regler (9900) an die Vielzahl von Steuergeräten angeschlossen ist, um die durch das jeweilige Steuergerät (5000, 7000, 9001-900N) gemessene Schwingungsfrequenz (Fmax1 int) zu empfangen,
und **wobei** die Vorrichtung zur Auswertung des Zustands der Spindeln (9901) dazu konfiguriert ist, die Spindeln, deren Schwingungsfrequenz im Intervall (INT) liegt, als gut zu bewerten,
**wobei** der Regler (9900) einen Chronometer (9904) umfasst und **wobei** die Vorrichtung zur Auswertung des Zustands der Spindeln (9901) dazu konfiguriert ist, die Spindeln, deren Schwingungsfrequenz eine vorbestimmte, durch den Chronometer (9904) gemessene Zeitspanne (TFTG) lang außerhalb des Intervalls (INT) liegt, als nicht gut zu bewerten und in der Lage ist, einen Alarm auszulösen.

2. Steuerungssystem nach Patentanspruch 1, **wobei** der optische Bewegungssensor (5801) einen optischen Generator (2501, 3511) umfasst, der dazu konfiguriert ist, eine modulierte Strahlung mit einer vorbestimmten Frequenz (F1) abzugeben.

3. Steuerungssystem nach Patentanspruch 2, **wobei** der Frequenzmesser (5802) einen Störfrequenzdetektor (7810) umfasst, der dazu konfiguriert ist, das Vorhandensein wenigstens einer Störfrequenz (F2, F3) in dem durch den optischen Bewegungssensor (5801) gemessenen Signal festzustellen.

4. Steuerungssystem nach Patentanspruch 3, **wobei** der Störfrequenzdetektor (7810) dazu konfiguriert ist, die Leistung (AIR1) der modulierten Strahlung und/oder die Verstärkung (AA1) des gemessenen Signals zu reduzieren und/oder zu erhöhen, wenn das Vorhandensein wenigstens einer Störfrequenz (F2, F3) festgestellt wird.

5. Steuerungssystem nach einem jeden der vorstehenden Patentansprüche, des Weiteren einen an den Frequenzmesser (5802) angeschlossenen Regler (2505) umfassend, **wobei** der Regler (2505) dazu konfiguriert ist, das Vorhandensein eines Problems zu erkennen, wenn die Schwingungsfrequenz (Fmax1_int) außerhalb des vorbestimmten Intervalls (INT) liegt.

## Revendications

1. Dispositif de contrôle (5000, 7000) pour machines à filer (1000), les machines à filer (1000) comprenant une pluralité de broches, chaque broche comprenant de premiers rouleaux de tirage (1101-1106) configurés pour tirer une première mèche (1201) de façon à obtenir un premier fil (1211, 1216, 1218),
le dispositif de contrôle (9000) comprenant une pluralité de dispositifs de contrôle (5000, 7000, 9001-900N) associables à une pluralité de broches, chaque dispositif de contrôle (5000, 7000, 9001-900N) comprenant :
- un capteur optique de mouvement (5801), et
- un fréquencemètre (5802) ;
**où** le capteur optique de mouvement (5801) est configuré de manière à mesurer le mouvement du premier fil (1211, 1216, 1218),
**où** le fréquencemètre (5802) est configuré de manière à recevoir le signal mesuré par le capteur optique de mouvement (5801) et de manière à mesurer au moins une fréquence d'oscillation (Fmax1_int) du premier fil (1211, 1216, 1218),
**caractérisé en ce que**
le système de contrôle (9000) comprenant en outre un contrôleur (9900), où le contrôleur comprend :
- un dispositif pour l'évaluation de la condition des broches (9901), configuré de manière à déterminer sur quels broches de la pluralité de broches le premier fil est présent et dans un état valide du fil, et
- un calculateur de valeur moyenne (9903) relié au dispositif pour l'évaluation de la condition des broches (9901), configuré de manière à calculer une valeur moyenne des rotations (MED) des broches sur lesquelles le premier file est présent et dans un état valide du fil, ou
- un capteur, configuré pour être positionné sur un moteur qui fait tourner les broches, configuré de manière à mesurer la valeur moyenne de rotations (MED) comme la valeur de rotations du moteur, et
- un calculateur d'intervalles (9902) configuré de manière à calculer un intervalle (INT) sur la base de la valeur moyenne de rotations (MED) et sur une valeur limite (SFTG),
**où** le calculateur d'intervalles (9902) est relié au dispositif pour l'évaluation de la condition des broches (9901) de manière à fournir au dispositif la valeur de l'intervalle (INT) pour l'évaluation de la condition des broches (9901),
**où** le contrôleur (9900) est relié à la pluralité des dispositifs de contrôle (5000, 7000, 9001-900N) de manière à recevoir la fréquence d'oscillation (Fmax1_int) mesurée par le dispositif de contrôle correspondant (5000, 7000, 9001-900N),
et **où** le dispositif pour l'évaluation de la condition des broches (9901) est configuré de manière à évaluer comme bonnes les broches dont la fréquence d'oscillation est comprise dans l'intervalle (INT),
**où** le contrôleur (9900) comprend un chronomètre (9904), et **où** le dispositif pour l'évaluation de la condition des broches (9901) est configuré de manière à évaluer comme non bonnes les broches dont la fréquence d'oscillation se trouve en dehors de l'intervalle (INT) pour une durée de temps prédéterminée (TFTG) mesurée par le chronomètre (9904) et de manière à être en mesure de déclencher une alarme.

2. Système de contrôle selon la revendication 1, **où** le capteur de mouvement optique (5801) comprend un générateur optique (2501, 3511) configuré de manière à émettre une radiation modulée avec une fréquence prédéterminée (F1).

3. Système de contrôle selon la revendication 2, **où** le fréquencemètre (5802) comprend un détecteur de fréquences simulées (7810) configuré de manière à déterminer la présence d'au moins une fréquence simulée (F2, F3) dans le signal mesuré par le capteur optique de mouvement (5801).

4. Système de contrôle selon la revendication 3, **où** le détecteur de fréquences simulées (7810) est configuré de manière à réduire et/ou à augmenter la puissance (AIR1) de la radiation modulée et/ou l'amplification (AA1) du signal mesuré si la présence d'au moins une fréquence simulée (F2, F3) est déterminée.

5. Système de contrôle selon l'une quelconque des revendications précédentes, comprenant en outre contrôleur (2505) relié au fréquencemètre (5802), **où** le contrôleur (2505) est configuré de manière à déterminer la présence d'un problème si la fréquence d'oscillation (Fmax1_int) se trouve en dehors de l'intervalle prédéterminé (INT).
